# EUROPEAN PATENT APPLICATION

(11) **EP 1 764 081 A1**
(43) Date of publication of application: **21.03.2007**
(21) Application number: 05291800.0
(22) Date of filing: 29.08.2005
(51) Int. Cl.: A61K 8/41, A61K 8/35, A61K 8/49, A61Q 17/04

(54) **Sunscreen composition comprising a dibenzoylmethane, an aminohydroxybenzophenone, a triazine and a triazole as UV filters**

(71) Applicant: Johnson & Johnson Consumer France SAS, 92787 Issy les Moulineaux Cedex 9 (FR)
(72) Inventor: Brillouet, Anne Sophie, 27400 Louviers (FR); Tischenbach, Isabelle, 27400 Louviers (FR); Dupressoir, Carole, 78300 Poissy (FR); Cole, Curtis A., Ringoes, New Jersey 08551 (US)
(74) Representative: Metten, Karl-Heinz

(57) **Abstract**

The present invention relates to a composition comprising: (a) a dibenzoylmethane derivative UV-A absorbing agent, (b) a hydroxybenzophenone, (c) a triazine derivative, and (d) a triazole derivative, and the topical use thereof in protecting mammalian skin or hair from UV radiation.

## Description

### FIELD OF THE INVENTION

The present invention relates to improved sunscreen compositions comprising (a) a dibenzoylmethane derivative UV-A absorbing agent, (b) a hydroxybenzophenone, (c) a triazine derivative, and (d) a triazole derivative. The compositions provide an excellent balance of UV-A and UV-B absorbance and good photostability.

### BACKGROUND OF THE INVENTION

The prolonged exposure to UV radiation, such as from the sun, can lead to the formation of light dermatoses and erythemas, as well as increase the risk of skin cancers, such as melanoma, and accelerate skin aging, such as loss of skin elasticity and wrinkling. Light having wavelengths in both the UV-A range (from about 320 to 400 nm) and the UV-B range (from about 280 to about 320 nm) can cause such skin damage, and, thus, sunscreen compositions should preferably comprise both UV-A and UV-B absorbers/reflectors (W sunscreens).

Numerous UV-B absorbers are available for sunscreening purposes, however there are far fewer choices available for UV-A filtration, particularly in the longer wavelength regions of the UV-A (340-400nm). Also hampering the efforts to provide high UV-A protection are strict regulations on the concentrations of these UV-A filters that can be included in sunscreen products.

It would desirable to have a sunscreen composition that provides both UV-A and UV-B protection using a minimum number of ingredients. In particular, if would be desirable to have a sunscreen composition that provides equal UV-A absorbance and UV-B absorbance. The present invention relates to the finding of an unexpected, advantageous effect between two classes of UV-A absorbing agents, which, when used together in certain ratios, provide compositions having excellent and balanced UV-A and UV-B absorbance, as well as good photostability.

### SUMMARY OF THE INVENTION

In one aspect, the invention features a composition comprising:
(a) a dibenzoylmethane derivative UV-A absorbing agent of the formula: 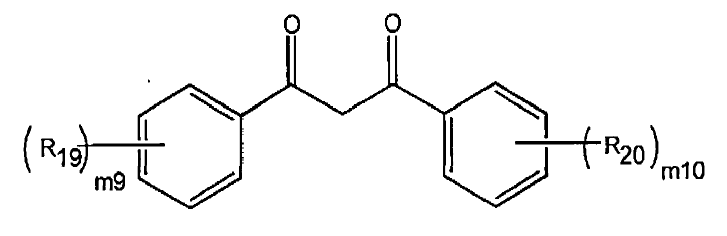 wherein R₁₉ and R₂₀, independently, are C₁-C₈ alkyl or C₁-C₈ alkoxy, m9 is 0 to 3, and m1 0 is 1 to 3;
(b) a hydroxybenzophenone of the formula: 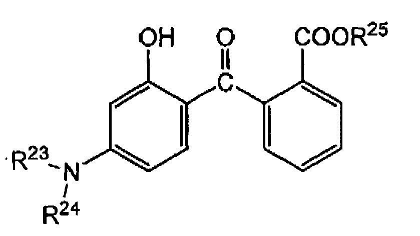 wherein R²³ and R²⁴ independently are hydrogen, C₁-C₁₀ alkyl, C₃-C₁₀ cycloalkyl, or C₃-C₁₀ cycloalkenyl, wherein the substituents R²³ and R²⁴, together with the nitrogen atom to which they bonded, can form a 5- or 6-membered ring; and R²⁵ is C₁-C₂₀-alkyl;
(c) a triazine derivative of the formula: 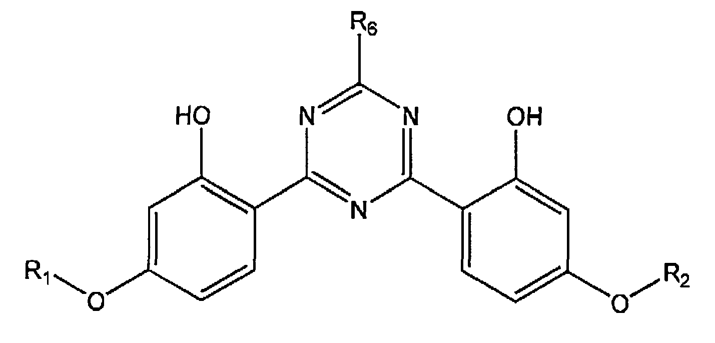 wherein, R₁ and R₂, independently, are C₃-C₁₈ alkyl, C₂-C₁₈ alkenyl, a radical of the formula -CH₂-CH(OH)-CH₂-O-R₈, or a radical of the formula (II) 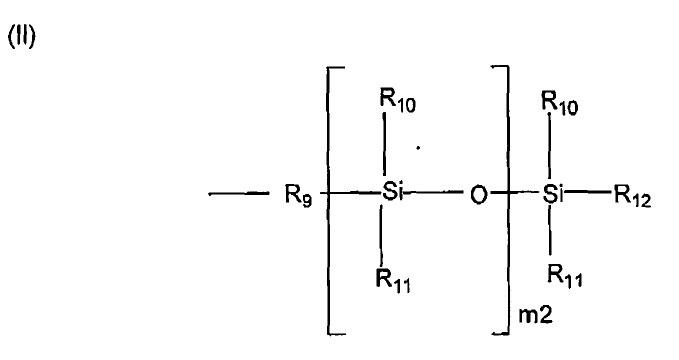 in which R₉ is a direct bond, C₁-C₄ alkylene, or a radical of the formula -Cₘ₁H₂ₘ₁- or -Cm₁H₂ₘ₁-O-; Rio, R₁₁, and R₁₂, independently, are C₁-C₁₈ alkyl, C₁-C₁₈ alkoxy,
   or a radical of the formula (III) 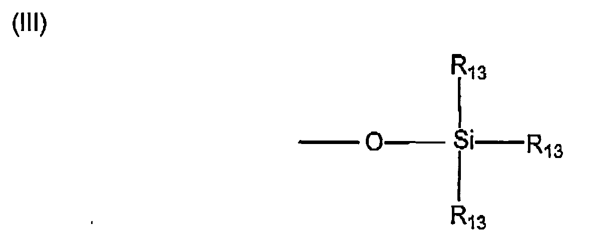 in which R₁₃ is C₁ -C₅ alkyl; m1 is 1 to 4; m2 is 0 to 5;
   R₆ is a radical of the formula (IV) 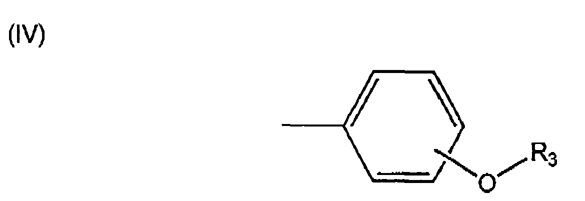 or of the formula (V) 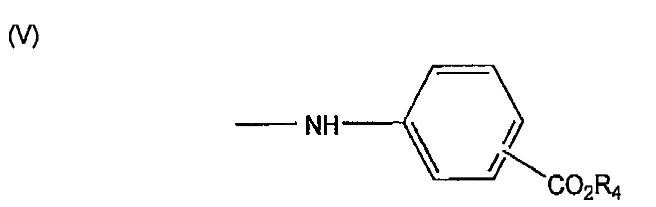 or of the formula (VI) 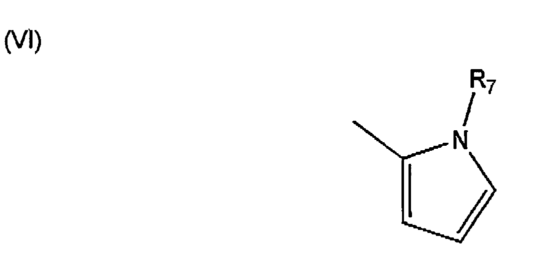 R₃ is hydrogen, C₁-C₁₀ alkyl, or a radical of the formula -(CH₂CHR₅-O)ₘ₄-CH₂-O-R₈ or -CH(OH)-CH₂-O-R₈;
   R₄ is hydrogen, a metal cation, C₁-C₅ alkyl, or a radical of the formula -(CH₂)ₘ₃-O-R₈;
   R₅ is hydrogen or methyl;
   R₈ is hydrogen or C₁-C₈ alkyl;
   R₇ is C₁-C₁₈ alkyl;
   m3 is 1 to 4; and
   m4 is 1 to 16;
   and
(d) a triazole derivative of the formula (VIII) or (IX): 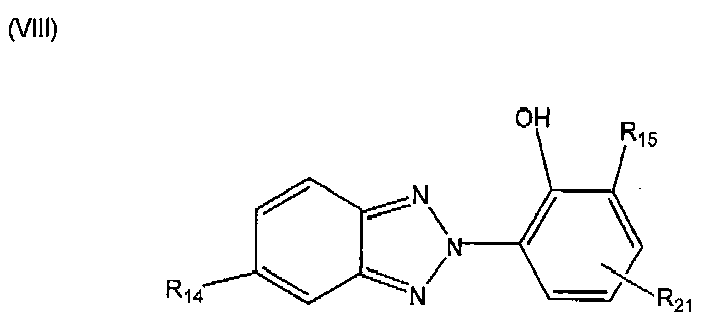 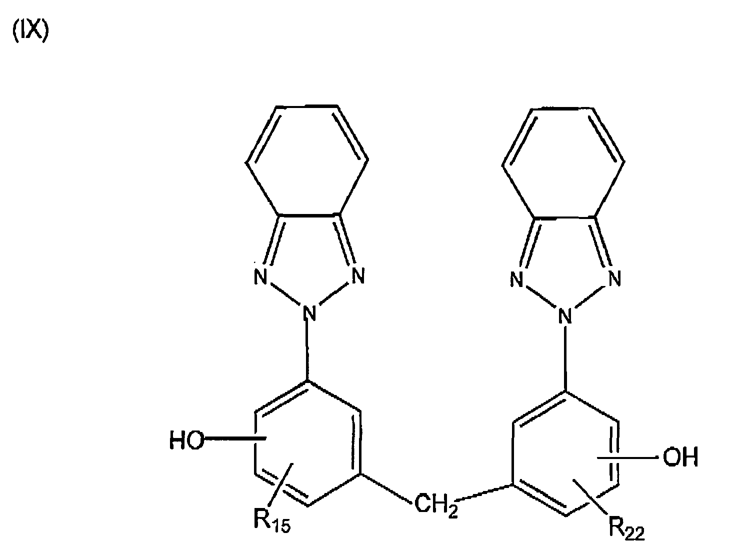
wherein R₁₄ is C₁-C₁₈ alkyl or hydrogen; R₁₅ and R₂₂, independently, are C₁-C₁₆ alkyl optionally substituted with a phenyl group, and R₂₁ is C₁-C₈ alkyl;
wherein said dibenzoylmethane derivative UV-A absorbing agent and said hydroxybenzophenone are present in a weight ratio ranging from about 1:3 to about 3:1.

The invention also features a method of protecting mammalian skin or hair from UV light comprising topically applying to the skin or hair the above composition.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. As used herein, unless otherwise indicated, all alkyl, alkenyl, and alkoxy groups may be straight or branched chain groups.

The composition comprises a dibenzoylmethane derivative UV-A absorbing agent. This is a compound comprising a dibenzoylmethane group and capable of absorbing radiation in the UV-A range (e.g., from about 320 to 400 nm). Examples of dibenzoylmethane derivative UV-A absorbing agents include those of the formula (VII): 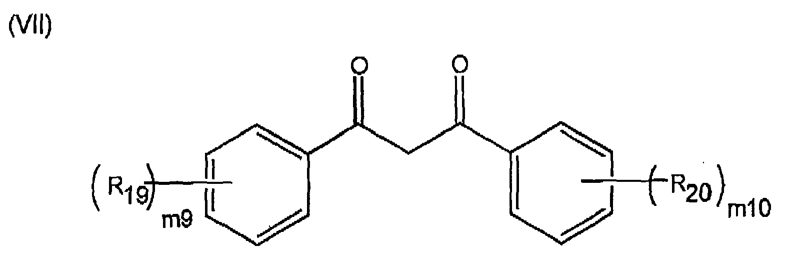 wherein R₁₉ and R₂₀, independently, are C₁-C₈ alkyl or C₁-C₈ alkoxy, m9 is 0 to 3, and m10 is 1 to 3. Examples and the synthesis of such compositions are disclosed in U.S. Patent No. 4,489,057 and include, but are not limited to, 4-(1,1-dimethylethyl)-4'-methoxydibenzoylmethane (avobenzone and sold as PARSOL 1789, Roche Vitamins and Fine Chemicals, Nutley, New Jersey, USA), 2- 2-methyldibenzoylmethane, 4- methyl-dibenzoylmethane, 4-isopropyldibenzoylmethane, 4-tert-butyldibenzoylmethane, 4-tert-butyl-4'-methoxydibenzoylmethane, 2,4-dimethylbenzoylmethane, 2,5-dimethylbenzoylmethane, 4,4'-diisopropylbenzoylmethane, 2-methyl-5-isopropyl-4'-methoxydibenzoylmethane, 2-methyl-5-tert-butyl-4'-methoxydibenzoylmethane, 2,4-dimethyl-4'- methoxydibenzoylmethane, and 2,6-dimethyl-4-tert-butyl-4'-methoxydibenzoylmethane. In one embodiment, the dibenzoylmethane derivative can range from about 0.1% to about 20%, by weight of the total composition (e.g., from about 1% to about 10%, by weight).

The composition also comprises a hydroxybenzophenone of the formula: 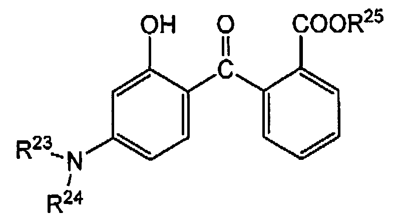 wherein R²³ and R²⁴ independently are hydrogen, C₁-C₁₀ alkyl, C₃-C₁₀ cycloalkyl, or C₃-C₁₀ cycloalkenyl, wherein the substituents R²³ and R²⁴, together with the nitrogen atom to which they bonded, can form a 5- or 6-membered ring; and R²⁵ is C₁-C₂₀-alkyl.

In one embodiment, the hydroxybenzophenone is 2-(4-diethylamino-2-hydroxybenzol)-benzoic acid hexylester, sold commercially as UNIVUL A PLUS from BASF Corporation:

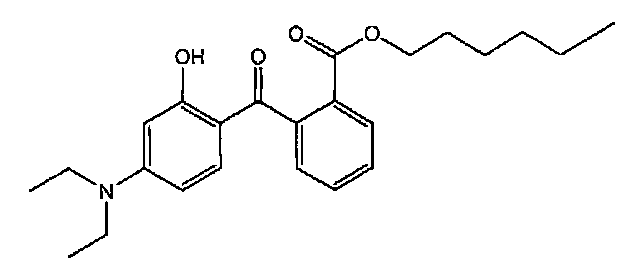

The composition further comprises a triazine derivative, which is a compound comprising one or more triazine group(s). In one embodiment, the triazine derivative is of the formula (1) 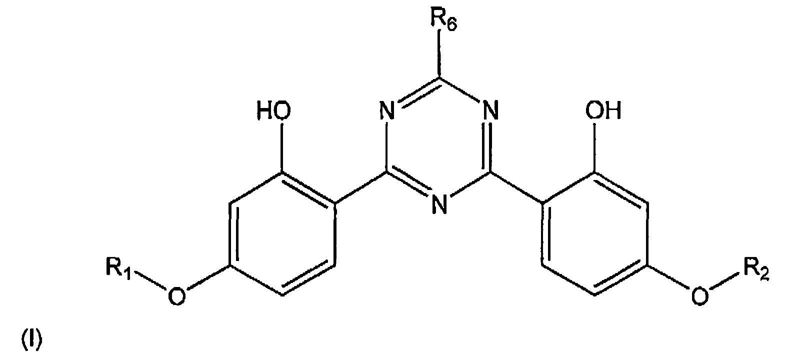 wherein,
R₁ and R₂, independently, are C₃-C₁₈ alkyl, C₂-C₁₈ alkenyl, a radical of the formula -CH₂-CH(OH)-CH₂-O-R₈, or a radical of the formula (II)

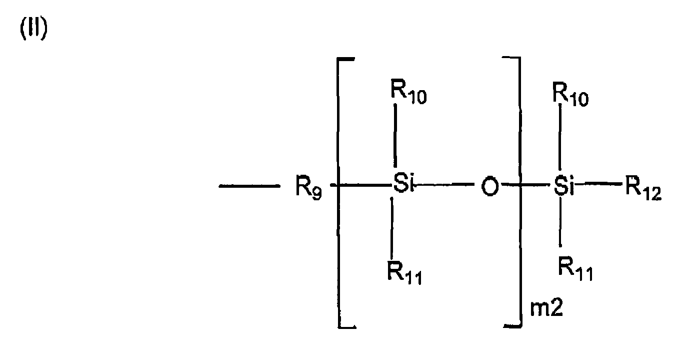

R₉ is a direct bond, C₁-C₄ alkylene, e.g. methylene, ethylene, propylene or butylene, or a radical of the formula -Cₘ₁H₂ₘ₁- or -Cₘ₁H₂ₘ₁-O-;
R₁₀, R₁₁, and R₁₂, independently, are C₁-C₁₈ alkyl, C₁-C₁₈ alkoxy, or a radical of the formula (III) 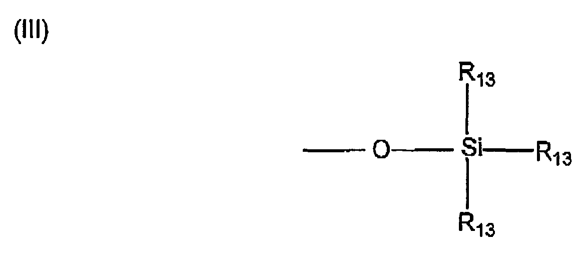 R₁₃ is C₁ -C₅ alkyl;
m1 is 1 to 4;
m2 is 0 to 5;
R₆ is a radical of the formula (IV) 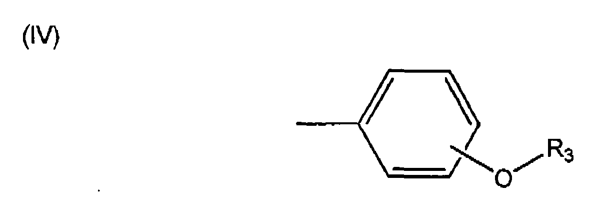 or of the formula (V) 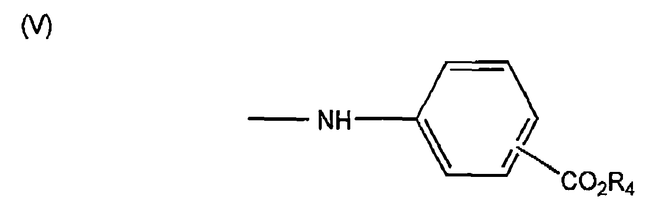 or of the formula (VI) 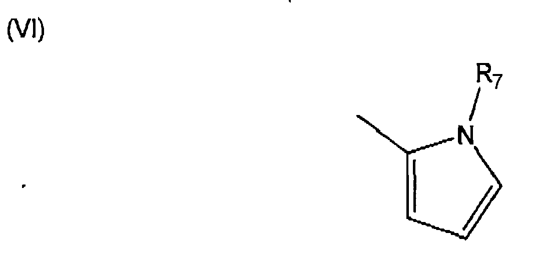 R₃ is hydrogen, C₁-C₁₀ alkyl, or a radical of the formula -(CH₂CHR₅-O)ₘ₄-(CH₂)_{q}CH₃ or -(CH₂CHR₅-O)ₘ₄-(CH₂)_{q}-O-R₈ or -CH(OH)-CH₂-O-R₈;
R₄ is hydrogen, a metal cation, C₁-C₅ alkyl, or a radical of the formula - (CH₂)ₘ₃-O-R₈;
R₅ is hydrogen or methyl;
R₈ is hydrogen or C₁-C₈ alkyl;
R₇ is C₁-C₁₈ alkyl;
m3 is 1 to 4;
m4 is 1 to 16; and
q is 0 to 16.

Examples of compounds of Formula (I), and the synthesis thereof, are described in U.S. Patent No. 5,955,060. In one embodiment, the compound of formula (I) is 2,4-Bis{[4-(2-ethyl-hexyloxy)-2-hydroxy]-phenyl}6-(4-methoxyphenyl)-(1,3,5)-triazine, sold commercially as TINOSORB S by Ciba Specialty Chemicals Corporation, Greensboro, North Carolina, USA.

The triazine derivative is present in an amount from about 0.1% to about 20%, by weight, of said composition (e.g., about 1% to about 10%, by weight, of the composition).

The composition also comprises a triazole derivative. This is a compound comprising one or more triazole groups. Examples of triazoles are compounds of the formula (VIII) or (IX): 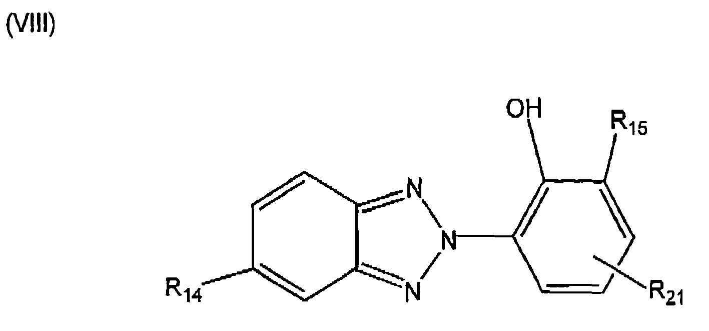 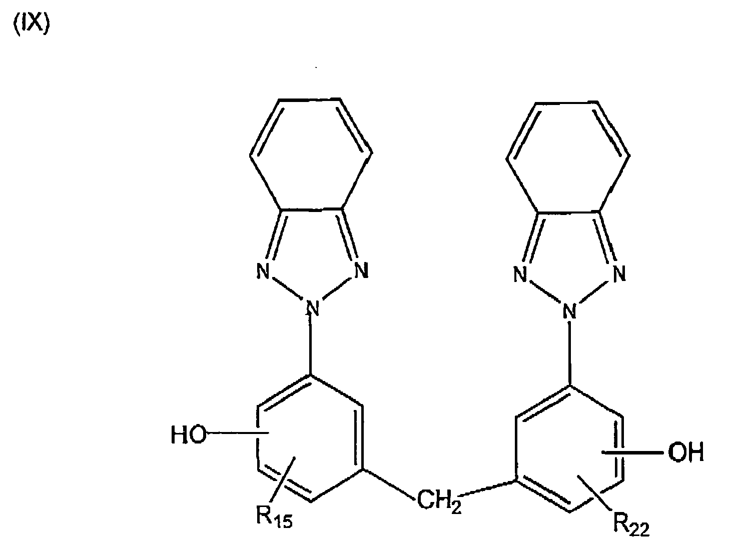 wherein R₁₄ is C₁-C₁₈ alkyl or hydrogen; R₁₅ and R₂₂, independently, are C₁-C₁₈ alkyl optionally substituted with a phenyl group, and R₂₁ is C₁-C₈ alkyl. Compounds of Formulae (VIII) and (IX) are described in U.S. Patent No. 5,869,030, and include, but are not limited to, methylene bis-benzotriazolyl tetramethylbutylphenol (TINSORB M, Ciba Specialty Chemicals Corporation, Greensboro, North Carolina, USA). In one embodiment, the triazole derivative can range from about 0.1% to about 20%, by weight, of the total composition (e.g., from about 1% to about 10%, by weight).

The weight ratio of the dibenzoylmethane derivative UV-A absorbing agent to the hydroxybenzophenone ranges from about 1:3 to about 3:1. In one embodiment, the weight ratio of dibenzoylmethane derivative UV-A absorbing agent to hydroxybenzophenone is about 1:1.

In another embodiment, the combined amount of said dibenzoylmethane derivative UV-A absorbing agent and said hydroxybenzophenone ranges from about 0.1% to about 20% by weight of the composition, preferably about 0.5% to about 5% by weight of the composition.

In another embodiment, the combined amount of the triazine derivative and the triazole derivative ranges from about 0.5% to about 7% by weight of the composition.

Importantly, the present composition provides balanced UV-A and UV-B protection. In one embodiment, the composition provides a ratio of UV-A absorbance to W-B absorbance of at least about 0.8, preferably at least about 0.9.

The ratio of UV-A to UV-B absorbance may be determined by the following *in vitro* method, which is used to define the Boots * Rating of the composition. Thin film spectroscopy of the composition is performed by applying the composition to a non-UV absorbing film or solid substrate such as methyl methacrylate in a density of around 0.75 to 2 mg/cm². The absorbance of the composition is measured in a UV spectrophotometer such as an Optometrics^{™} (UK) or Labsphere^{™} (NH) spectrophotometer to determine the absorbance throughout the UV spectrum, 290 - 400nm. The ratio of the average absorbance in the UV-A range (320-400nm) divided by the mean of the absorbance in the UV-B range is calculated to determine the "flatness" of the spectral absorbance of the composition. Values approaching 1 are given the highest Boots * Rating indicating "balanced protection" between both portions of the ultraviolet spectrum:

| Ratio UV-A/UV-B | BOOTS STARS | Category Descriptor |
|---|---|---|
| 0 to 0.19 | - | Too low for UVA Claim |
| 0.20 to 0.39 | * | MINIMUM |
| 0.40 to 0.59 | ** | MODERATE |
| 0.60 to 0.79 | *** | GOOD |
| 0.80 to 0.90 | **** | SUPERIOR |
| 0.90< | ***** | ULTRA |

In particular, the present composition provides a Boots * Rating of at least 3, preferably at least 4, more preferably 5.

The *in vivo* ratio of UV-A absorbance to UV-B absorbance may also be calculated. *In vivo* UV-B absorbance (SPF) of a composition is determined on human volunteers using a standard sunscreen testing protocol (the "Colipa Method"). Briefly, the minimum dose of solar-simulated ultraviolet radiation (UVR) required to induce a minimally perceptible erythema on human skin is determined for untreated skin and for the skin treated with the composition (erythema readings taken 24 hours after irradiation). The ratio of the dose of UV radiation needed to induce minimally perceptible erythema for the composition-protected skin (MEDp), divided by the dose required for a minimally perceptible erythema for unprotected skin (MEDu) results in the SPF value of the composition.

An irradiation apparatus used for SPF determinations is, for example, a Multiport Solar Simulator Model 601 (Solar Light Co., Philadelphia, Pennsylvania, USA) which consists of a 300 W Xenon lamp filtered with a UG11 1 mm thick filter and a WG320 1 mm filter (Schott Co., Philadelphia, Pennsylvania, USA) to allow exposure to UV between 240 and 800 nanometers.

*In vivo* determination of the UV-A protectiveness of the composition is determined using the PPD test protocol. The procedure is similar to the SPF test except that the PPD test uses only the longer portion of the UV spectrum (from 320 to 400nm) by using UG11 1mm thick and WG335 3mm thick filters (Schott Co.). Also, the biological reaction measured is not erythema as for the SPF determination, but rather pigmentation examined 2 hours after exposure ("persistent pigment darkening" or "PPD"). The PPD protection factor is the ratio of the UV-A dose required to induce minimally perceptible pigment darkening for formulation protected skin (MPPDp), divided by the UV-A dose required for unprotected skin (MPPDu).

Accordingly, the *in vivo* UV-A absorbance/UV-B absorption ratio is then calculated as the ratio of PPD protection factor/SPF value.

In one embodiment, the composition simultaneously provides a high SPF and high critical wavelength. For example, the composition comprises an SPF of at least about 15, preferably at least about 18, more preferably at least about 20, and a critical wavelength of at least about 370. In another embodiment, the composition provides an SPF of at least about 15, preferably at least about 18, more preferably at least about 20, a critical wavelength of at least about 370, and a PFA of at least about 10.

PFA is biological protection factor in the UV-A spectrum. The PFA can be determined *in vivo,* using for example the above PPD protection factor method, see also Cole (1991), or can be estimated using *in vitro* test models.

The composition also provides good photostability.

In one embodiment, the composition further comprises one or more additional UV-A and/or UV-B absorbing/reflecting agent(s). Examples of such absorbing/reflecting agents include, but are not limited to: methoxycinnamate derivatives such as octyl methoxycinnamate and isoamyl methoxycinnamate; camphor derivatives such as 4-methyl benzylidene camphor, camphor benzalkonium methosulfate, and terephthalylidene dicamphor sulfonic acid; salicylate derivatives such as octyl salicylate, trolamine salicylate, and homosalate; sulfonic acid derivatives such as phenylbenzimidazole sulfonic acid; benzone derivatives such as dioxybenzone, sulisobenzone, and oxybenzone; benzoic acid derivatives such as aminobenzoic acid and octyldimethyl para-amino benzoic acid; octocrylene and other β,β-diphenylacrylates; dioctyl butamido triazone; titanium dioxide, zinc oxide; iron oxides; octyl triazone; butyl methoxydibenzoyl methane; drometrizole trisiloxane; and menthyl anthranilate. Other UV absorbers/reflectors useful herein can be found in Sagarin, Cosmetics Science and Technology, Chapter VIII, pages 189 et seq. and the *ICI Handbook* page 1672. A list of such compounds is also disclosed in U.S. Patent Number 4,919,934.

The individual UV absorbing/reflecting agent concentration can range from about 0.1 % to about 30%, by weight, of the composition (e.g., from about 1 % to about 20%, by weight). The total concentration of all such agents should be based on the desired sunscreen protection factor ("SPF") level (e.g., an SPF level of from about 10 to about 60).

In one embodiment, the composition additionally comprises octocrylene or another β,β-diphenylacrylate.

In another embodiment, the composition further comprises a diester or polyester of a naphthalene dicarboxylic acid. Examples of diesters and polyesters of a naphthalene dicarboxylic acid are compounds of formulae (X) or (XI): 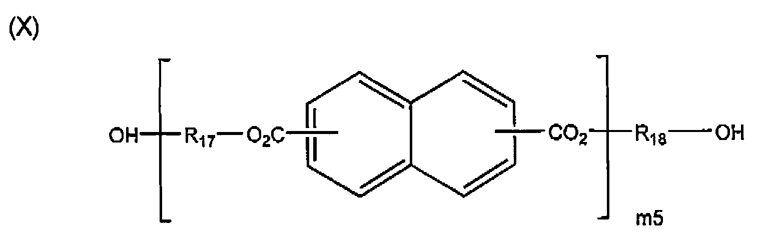 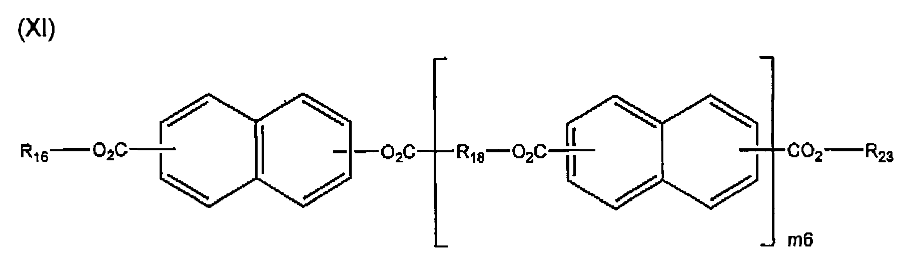 wherein R₁₆ and R₂₃, independently, are selected from the group consisting of a C₁-C₂₂ alkyl, a diol having the structure HO-R₁₈-OH, and a polyglycol having the structure HO-R₁₇-(-O-R₁₈-)ₘ₅-OH; R₁₇ and R₁₈, independently, are C₁-C₆ alkenyl; and m5 and m6, independently, are each in the range of 1 to about 100. Examples, including the synthesis, of such diesters or polyesters of naphthalene dicarboxylic acid are described in U.S. Patent No. 5,993,789, and include, but not limited to, diethylhexyl naphthalate (HALLBRITE TQ, C.P. Hall Company, Bedford Park, Illinois, USA). See Bonda, et al., Allured's Cosmetic & Toiletries Magazine, 115(6):37-45 (2000) disclosing the uses of such compounds in sunscreen compositions. In one embodiment, the diester or polyester of a naphthalene dicarboxylic acid can range from about 0.1% to about 30%, by weight, of the total composition (e.g., from about 1% to about 10%, by weight).

In one embodiment, the composition further comprises an alkyl benzoate derivative. Examples of alkyl benzoate derivatives are compounds of the formulae (XII) or (XIII), 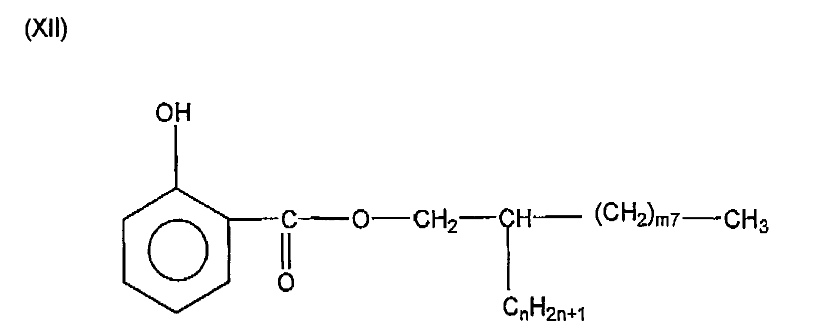 wherein m7 is 5, 7, or 9 and n is 4, 6, or 8; 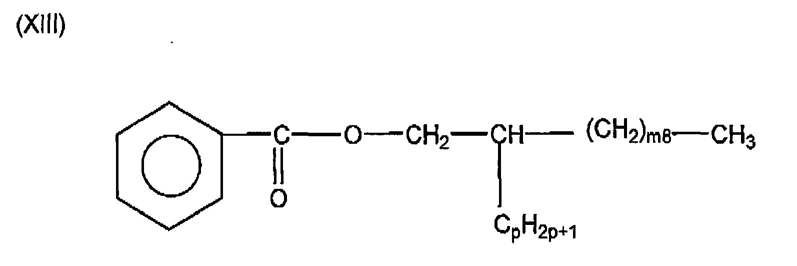 wherein m8 is 5 or 7 and p is 4 or 6.

The compounds of formulae (XII) and (XIII) may be formed by typical esterification and transesterification reactions, e.g., as describe in U.S. Patent No. 5,783,173. Examples of such long branched chain alkyl benzoates are listed in U.S. Patent No. 5,783,173 and include, but not limited to, butyloctyl salicylate (HALLBRITE BHB, C.P. Hall Company, Bedford Park, Illinois, USA). In one embodiment, the alkyl benzoate derivative can range from about 0.1 % to about 30%, by weight, of the total composition (e.g., from about 1% to about 10%, by weight).

The compositions of the present invention may further comprise one or more other cosmetically active agent(s). A "cosmetically active agent" is a compound that has a cosmetic or therapeutic effect on the skin, e.g., agents to treat wrinkles, acne, or to lighten the skin. In one embodiment, the agent is selected from, but not limited to, the group consisting of: hydroxy acids; benzoyl peroxide; sulfur resorcinol; D-panthenol; hydroquinone; antiinflammatory agents; skin lightening agents; antimicrobial and antifungal agents such a miconazole, ketoconazole, and elubial; vitamins such as ascorbic acid; tocopherols and tocotrienols such as tocopheryl acetate; retinoids such retinol, retinal, retinyl palmitate, retinyl acetate, and retinoic acid; hormones such as estrogens and dihydroxyandrostene dione; 2-dimethylaminoethanol; lipoic acid; amino acids such a proline and tyrosine; lactobionic acid; self-tanning agents such as dihydroxy acetone; dimethyl aminoethanol; acetyl-coenzyme A; niacin; riboflavin; thiamin; ribose; electron transporters such as NADH and FADH2; botanical extracts such as ginkgo biloba, aloe vera, and soy; and derivatives thereof. The cosmetically active agent will typically be present in the composition of the invention in an amount of from about 0.001% to about 20% by weight of the composition, e.g., about 0.01% to about 10% such as about 0.1% to about 5% by weight of the composition.

Examples of hydroxy acids include, but are not limited, to (i) alpha-hydroxy acids such as glycolic acid, lactic acid, malic acid, citric acid, and tartaric acid, (ii) beta-hydroxy acids such as salicylic acid, and/or (iii) polyhydroxy acids. See, e.g., European Patent Application No. 273,202.

Examples of derivatives of ascorbic acid include, but are not limited to, ascorbyl palmitate, magnesium ascorbyl phosphate, sodium ascorbyl phosphate, zinc ascorbyl phosphate, ascorbyl glucoside, sodium ascorbate, and ascorbyl polypeptide. An example of a derivative of hydroquinone includes, but is not limited to, arbutin.

The compositions of the present invention may also comprise one or more of the following: antioxidants (e.g., ascorbic acid, tocopherols, polyphenols, tocotrienols, BHA, and BHT), chelating agents (e.g., EDTA), and preservatives (e.g., parabens). Examples of suitable antioxidants, preservatives, and chelating agents are listed in pp. 1612-13, 1626, and 1654-55 of the *ICI Handbook.* In addition, the topical compositions useful herein can contain conventional cosmetic adjuvants, such as dyes, opacifiers (e.g., titanium dioxide), pigments, and fragrances.

The compositions of the present invention can be used by topically administering to a mammal, e.g., by the direct laying on or spreading of the composition on the skin or hair of a human. The compositions useful in the subject invention, thus, involve formulations suitable for topical application to mammalian skin, the formulation comprising (i) a dibenzoylmethane derivative UV-A absorbing agent, (ii) a hydroxybenzophenone, (iii) a triazine derivative, (iv) a triazole derivative, (v) optionally, other compounds/agents such as other UV-A and UV-B reflectors/absorbers listed herein, and (iv) a cosmetically-acceptable topical carrier. The term "cosmetically-acceptable topical carrier" refers to a carrier for topical use that is capable of having the other ingredients dispersed or dissolved therein, and possessing acceptable safety properties.

The topical compositions useful in the present invention may be used for a variety of cosmetic uses, including, but not limited to, protection the skin or hair from UV radiation. The compositions, thus, may be made into a wide variety of product types. These include, but are not limited to lotions, creams, gels, sticks, sprays, ointments, mousses, and cosmetics/make-up. These products may comprise several types of carrier systems including, but not limited to single phase solutions (e.g., oil based solutions), emulsions, and gels.

The compositions of the present invention may be prepared using methodology that is well known by an artisan of ordinary skill.

### Example 1

The following compositions according to the invention were prepared. They contained UNIVUL A Plus, PARSOL 1789, TINOSORB M and TINOSORB S, as shown in Table 1 below. The compositions also contained 0.8 weight % octocrylene and 1.5 weight % ethylhexyl triazine, along with various conventional sunscreen additives.

The Boots * Rating and SPF (as described above) were measured for each composition. The results are shown in Table 1 below.

**Table 1**

| Composition | Uvinul A Plus (weight %) | Parsol 1789 (weight %) | Ratio Uvinul A Plus/Parsol 1789 | Tinsorb M (weight %) | Tinsorb S (weigh %) | SPF | Soots * Rating |
|---|---|---|---|---|---|---|---|
| 257-090 | 2% | 1% | 2/1 | 5% | 2% | 71.8 | 4(0.86) |
| 257-070 | 3% | 1% | 3/1 | 5% | 2% | - | 4 (0.87) |
| 257-134 | 2% | 2% | 1:1 | 5% | 1% | 57.8 | 5 (0.92) |

### Example 2

Three compositions according to the invention were prepared containing a 1:1 ratio of UVINUL A Plus to PARSOL 1789, along with TINSORB S, TINOSORB M, and 5 weight % octocrylene. The SPF was varied between 20 and 50+ by adjusting the amounts of UV filters, and in the case of the SPF 50+ composition, 3 weight % EUSOLEX TS (titanium dioxide) was added. Each had a Boots *Rating of 5 and an *in vivo* UV-A absorbance/UV-B absorbance ratio of greater than 1/3. The results are shown in Table 2.

**Table 2**

| | SPF 20 | SPF40 | SPF50+ |
|---|---|---|---|
| % Uvinul A Plus (weight %) | 1.5 | 2 | 2 |
| %Parsol 1789 (weight %) | 1.5 | 2 | 2 |
| Boots * Rating | 0.92 (5*) | 0.96 (5*) | 0.96 (5*) |
| *In-vivo* ratio | 42%>1/3 | 81%>>1/3 | 38%>1/3 |
| PhotostabilityUVA | 91% | | |
| Photostability UVB | 100% | 95% | 98% |

UV-A and UV-B photostability were measured using a UV/visible model 752 spectroradiometer (OPTRONIC Laboratory), which consists of an external sphere, a double monochromator and a photomultiplier, controlled by a microprocessor. The lighting system was provided by an unfiltered 75 watt OSRAM Xenon lamp. Roughened quartz plates were used as the substrate (70mm x 70mm x 1mm from THUET-BIECHELIN LABS, Blodelsheim, France). In each case, transmission of the quartz plate was first assessed each 5 nm before applying the composition. Then, 0.75 mg/cm² of composition was applied to the quartz plate using a micro-syringe. The plate was then left half an hour in the dark at room temperature to ensure self-leveling of the composition before measuring transmission each 5 nm. The composition was then irradiated with 10 MED and transmission was measured again. Photostability was assessed by determining the loss in efficacy (as a percentage) of the tested composition in the UVB and in the UVA ranges (calculation of the SPF or PFA before and after irradiation).

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

**1.** A composition comprising:
(a) a dibenzoylmethane derivative UV-A absorbing agent of the formula: 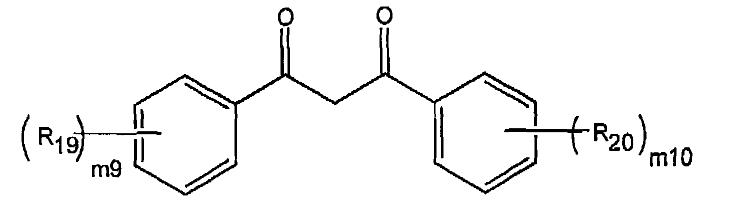 wherein R₁₉ and R₂₀, independently, are C₁-C₈ alkyl or C₁-C₈ alkoxy, m9 is 0 to 3, and m10 is 1 to 3;
(b) a hydroxybenzophenone of the formula: 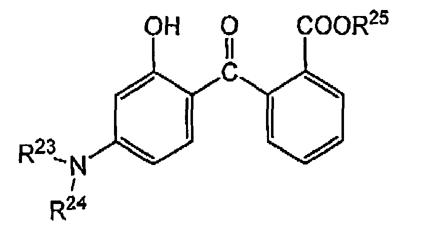 wherein R²³ and R²⁴ independently are hydrogen, C₁-C₁₀ alkyl, C₃-C₁₀ cycloalkyl, or C₃-C₁₀ cycloalkenyl, wherein the substituents R²³ and R²⁴, together with the nitrogen atom to which they are bonded, can form a 5- or 6-membered ring; and R²⁵ is C₁-C₂₀-alkyl;
(c) a triazine derivative of the formula: 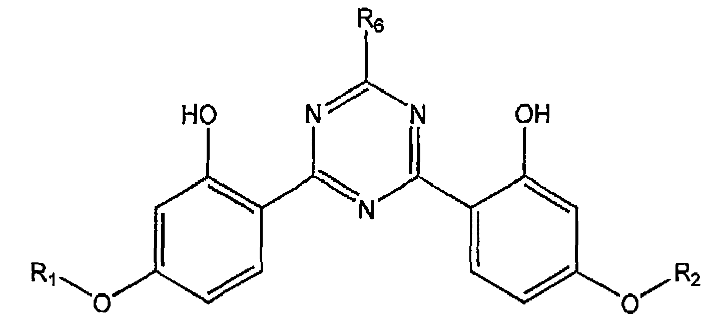 wherein, R₁ and R₂, independently, are C₃-C₁₈ alkyl, C₂C₁₈ alkenyl, a radical of the formula -CH₂-CH(OH)-CH₂-O-R₈, or a radical of the formula (II) 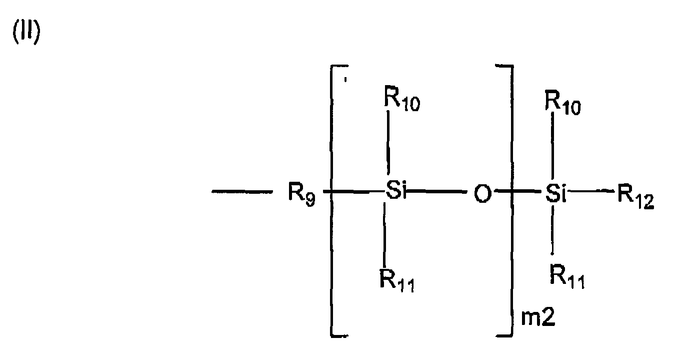 in which R₉ is a direct bond, C₁-C₄ alkenyl, or a radical of the formula -Cₘ₁H₂ₘ₁- or -Cₘ₁H₂ₘ₁-O-; R₁₀, R₁₁, and R₁₂, independently, are C₁-C₁₈ alkyl, C₁-C₁₈ alkoxy, or a radical of the formula (III) 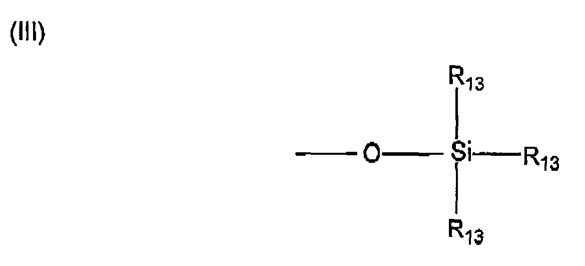 in which R₁₃ is C₁ -C₅ alkyl; m1 is 1 to 4; m2 is 0 to 5;
R₆ is a radical of the formula (IV) 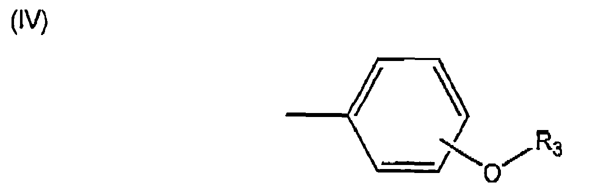 or of the formula (V) 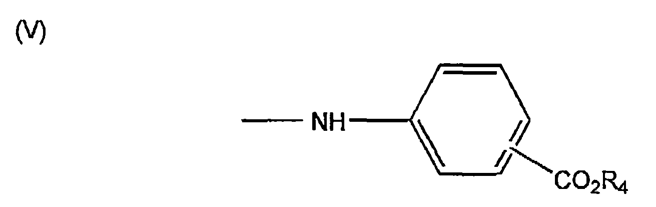 or of the formula (VI) 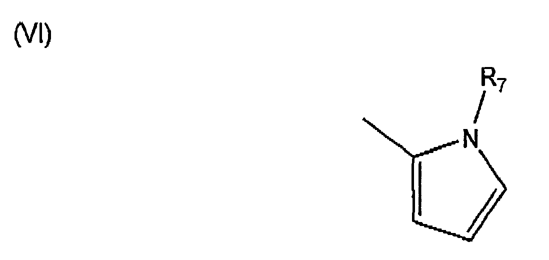 R₃ is hydrogen, C₁-C₁₀ alkyl, or a radical of the formula -(CH₂CHR₅-O)ₘ₄-R₄-CH₂ or -CH(OH)-CH₂-O-R₈;
R₄ is hydrogen, a metal cation, C₁-C₅ alkyl, or a radical of the formula -(CH₂)ₘ₃-O-R₈;
R₅ is hydrogen or methyl;
R₈ is hydrogen or C₁-C₈ alkyl;
R₇ is C₁-C₁₈ alkyl;
m3 is 1 to 4; and
m4 is 1 to 16;
and
(d) a triazole derivative of the formula (VIII) or (IX): 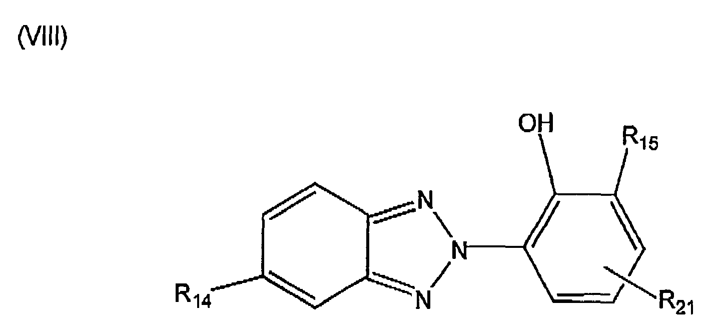 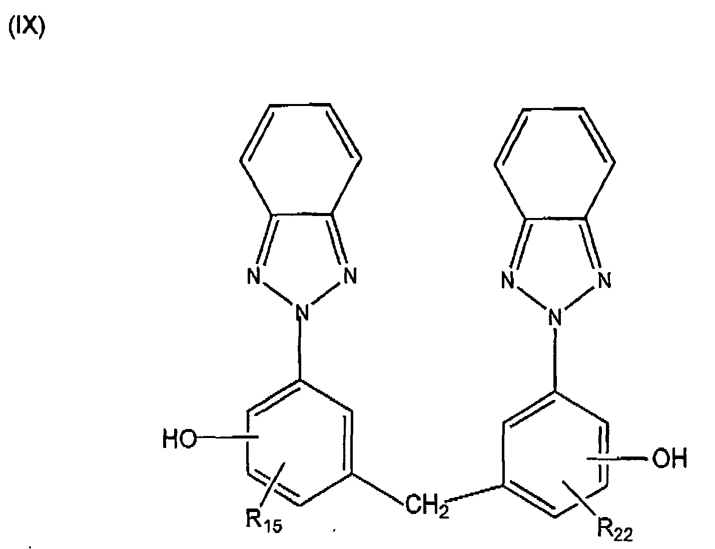
wherein R₁₄ is C₁-C₁₈ alkyl or hydrogen; R₁₅ and R₂₂, independently, are C₁-C₁₈ alkyl optionally substituted with a phenyl group, and R₂₁ is C₁-C₈ alkyl;
wherein said dibenzoylmethane derivative UV-A absorbing agent and said hydroxybenzophenone are present in a weight ratio ranging from about 1:3 to about 3:1.

**2.** The composition of claim 1 wherein said triazine derivative is 2,4-bis{[4-(2-ethyl-hexyloxy)-2-hydroxy]-phenyl}-6-(4-methoxyphenyl)-(1,3,5)-triazine.

**3.** The composition of claim 1, wherein said triazole derivative is methylene bis-benzotriazolyl tetramethylbutylphenol.

**4.** The composition of claim 1, wherein said hydroxybenzophenone is 2-(4-diethylamino-2-hydroxybenzol)-benzoic acid hexylester.

**5.** The composition of claim 1, wherein said dibenzoylmethane derivative UV-A absorbing agent is 4-tert-butyl-4'-methoxydibenzoylmethane.

**6.** The composition of claim 1, wherein the weight ratio of dibenzoylmethane derivative UV-A absorbing agent to hydroxybenzophenone is about 1:1.

**7.** The composition of claim 1, wherein the combined amount of said dibenzoylmethane derivative UV-A absorbing agent and said hydroxybenzophenone ranges from about 0.1 % to about 20% by weight of said composition.

**8.** The composition of claim 1, wherein the combined amount of said dibenzoylmethane derivative UV-A absorbing agent and said hydroxybenzophenone ranges from about 0.5% to about 5 % by weight of said composition.

**9.** The composition of claim 1 further comprising one or more UV-B absorbing agents.

**10.** The composition of claim 9, wherein said UV-B absorbing agent is selected from the group consisting of methyl benzylidene camphor, octyl salicylate, phenylbenzimidazole sulfonic acid, homosalate, zinc oxide, dioxybenzone, sulisobenzone, oxybenzone, octocrylene, and dioctyl butamido triazone.

**11.** The composition of claim 9, wherein said UV-B absorbing agent is present in an amount from about 0.1 % to about 20% by weight of said composition.

**12.** The composition of claim 1, wherein the combined amount of said triazine derivative and said triazole derivative ranges from about 0.5% to about 7% by weight of said composition.

**13.** The composition of claim 1, which provides a ratio of UV-A absorbance to UV-B absorbance of at least about 0.8. 14, The composition of claim 1, which provides a ratio of UV-A absorbance to UV-B absorbance of at least about 0.9.

**15.** The composition of claim 1, comprising an SPF of at least about 15 and a critical wavelength of at least about 370.

**16.** The composition of claim 1, comprising an SPF of at least about 15, a critical wavelength of at least about 370, and a PFA of at least about 10.

**17.** A method of protecting mammalian skin or hair from UV radiation comprising topically applying to the skin or hair a composition of claim 1.
